# EUROPEAN PATENT SPECIFICATION

(11) **EP 2 392 302 B1**
(45) Date of publication and mention of the grant of the patent: **13.02.2013**
(21) Application number: 11175725.8
(22) Date of filing: 28.05.2003
(51) Int. Cl.: A61F 13/53, A61B 17/88, A61H 9/00

(54) **Directed tissue growth employing reduced pressure**
Gezieltes Gewebewachstum unter Anwendung von verringertem Druck
Croissance de tissus dirigée avec une pression réduite

(30) Priority: 03.06.2002 US 161076
(43) Date of publication of application: 07.12.2011
(62) Divisional of application: 03756228.7
(73) Proprietor: Wake Forest University Health Sciences, Winston-Salem, NC 27157 (US)
(72) Inventor: ARGENTA, Louis, C., Winston-Salem, NC North Carolina 27104 (US); MORYKWAS, Michael, J., Winston-Salem NC 27104 (US); WEBB, Lawrence, X., Winston-Salem, NC North Carolina 27106 (US)
(74) Representative: Gill, Stephen Charles

(56) References cited:
- EP-A- 1 064 958
- WO-A-94/20041
- WO-A-99/51164
- US-A1- 2001 043 943

## Description

### Field of the Invention

The present invention relates to an apparatus and method for promoting directed tissue growth, and more particularly to an apparatus and method for providing directed tissue growth within a host matrix through the application of reduced pressure to the tissue to be grown.

### Background of the Invention

Promoting the growth of tissue, especially tissue damaged through trauma or disease, has long been an area of concern in medical practice. Such damage or disease, including complications due to infection, may hinder or prevent healing of an injury due to a lack of healthy tissue growth. Many diseases and certain injuries involve affected tissue that cannot heal spontaneously. Such is the case, for example, for an open pilon fracture of bone tissue. Historically, a pilon fracture involves a high complication rate. Such complications include infection, nonunion, failure to obtain or maintain a reduction of the joint surface, and early and late arthritis. Under such conditions, failure to achieve sufficient healing of the pilon fracture ,could necessitate amputation.

In the 1970s and early 1980s the prescribed treatment for most pilon fracture injuries was open reduction and internal fixation, usually with a metaphysical bone graft. Reports of high complication rates with this approach prompted many surgeons to use indirect methods such as bridging external fixation and to limit the surgery to what was necessary for the joint reduction. Awareness of the issues of timing has prompted some to use a staged procedure, with bridging external fixation initially, followed by open but limited surgery. The incisions are dictated by fracture patterns, and the timing is dictated by resolution of the soft tissue envelope.

However, despite these approaches, cases arise where a major complication, e.g., a deep infection, can develop. Depending on the patient's medical condition, such as the condition of local blood vessels, customary treatment by application of a free muscle flap may be inappropriate. In such instances, traditional treatment offers a poor prognosis for salvage of the affected tissue. In such cases, where there is a likelihood of an infected nonunion and its associated pain, deformity and poor function, amputation is the appropriate and preferred medical treatment. Thus, it could be a great advance to the medical practice to provide an apparatus and method to promote healthy bone tissue growth under such circumstances to avoid the drastic treatment of amputation.

As further example, diseases such as cancer often result in tissue damage that does not heal spontaneously, and treatment of such resulting tissue damage would benefit from an apparatus and method to promote tissue growth. For example, many patients who experience injuries or suffer from bone cancer require replacement of a missing piece of bone. Current techniques for bone replacement include: moving a piece of the bone from an uninjured site to the injured site; use of cadaver bone; or the use of metal rods or plates. These options are not always possible due to the potential for defect from the bone donor site, or the lack of availability of cadaver bone. Hence, growth of new healthy bone tissue would provide a valuable treatment option in such cases.

In addition to growth of bone tissue, growth of other body tissues such as cartilage, skin, tendon, nerves, breast-tissue, and organs such as the liver or pancreas, would provide a valuable advance in the medical practice. Many diseases exist which damage the tissue of an organ beyond the ability of the body to naturally repair such damage. For example, chronic injury to the liver through viral infection or other causes can ultimately lead to cirrhosis of the liver. As cirrhosis progresses healthy tissue is replaced with fibrous tissue. The blood vessels thicken and their channels may become obliterated, which reduces blood flow in the organ. The normal structure of the internal tissue is lost, and only nonfunctioning scar tissue remains. The lack of healthy tissue eventually leads to death. It would be a great advance to promote growth of remaining healthy tissue in the liver in combination with treatment for the underlying cause of the cirrhosis.

Moreover, many other diseases exist which are caused by damage to tissues of an organ, of which, diabetes is one of pressing importance. Presently, the number of individuals with diabetes doubles every 15 years. While insulin treatment can prevent early death from diabetic coma, such treatment does not prevent the chronic, disabling complications of the disease. Currently, diabetes mellitus is among the top 10 causes of death in the United States, and is the leading cause of blindness and uremia.

The first type of diabetes, Type I, is caused by failure of the pancreas to secrete insulin. Normally, insulin is synthesized in the beta cells of the islets of Langerhans of the pancreas. Individuals affected with Type I diabetes have insulin deficiency due to islet cell loss. Recent medical studies have shown the transplantation of cadaver beta cells to a diabetic patient can provide the pancreas with the ability to produce insulin. However, such an approach is limited due to the lack of availability of cadaver donor cells, the need for subsequent transplants, as well as complications and side effects commonly encountered in transplantation, such as the need for continued use of antirejection drugs. The ability to provide directed tissue growth of beta cells within the pancreas, or externally to the pancreas for transplantation into the pancreas, would therefore be a significant advance the treatment of diseases such as diabetes.

### Summary of the Invention

In accordance with the present invention a directed tissue growth apparatus is provided for growing tissue by applying a tissue growth medium to the tissue and applying a reduced, sub-atmospheric pressure at the growth medium and the tissue in a controlled manner for a selected time period. The application of reduced pressure at the growth medium and tissue promotes growth of the tissue within the tissue growth medium, which provides benefits such as accelerated healing rates and replacement of missing, diseased, or damaged tissue. Tissues that may exhibit a positive response to treatment by the application of reduced pressure include bone, cartilage, tendon, nerves, skin, breast tissue, and organs such as the liver or pancreas, for example.

The directed tissue growth apparatus in accordance with the present invention includes a reduced pressure application appliance which is applied to a treatment site where tissue is to be grown. Such a treatment site may be located in vivo or in vitro. The reduced pressure application appliance may include a tissue growth medium for placement in contact with the tissue to be grown to provide a medium into which cells may be grown. The form of the tissue growth medium is selected with regard to the type of tissue to be grown. For example, the tissue growth medium may comprise a layer of material suitable for use as artificial skin to replace damaged or missing skin. In such a case, the tissue growth medium may conveniently comprise a bioabsorbable material. A bioabsorbable material is a material that may dissolve in the tissue or which may be incorporated in the tissue as a substantially indistinguishable component. The tissue growth medium may also comprise a porous scaffold or matrix, which may include one or more of a naturally occurring fibrous or fibrous-proteinaceous material, such as collagen, or a synthetic resorbable material. For example, the scaffold may comprise a bone substitute material, such as a bioglass or ceramic. Porous materials permit growth of cells within the pores of the material and permit gas flow to the tissue during times of non-application of reduced pressure. In addition, during times when the sub-atmospheric pressure is applied to such porous materials, the porous materials facilitate distribution of sub-atmospheric pressure and fluid flow.

Optionally, an open-cell foam section, for connection to a source of reduced pressure, may be provided in contact with a selected tissue growth medium to apply a reduced pressure to the tissue growth medium. For example, the open-cell foam section may desirably be used with a particular tissue growth medium such as artificial skin, to better apply and distribute sub-atmospheric pressure across the skin surface.

The appliance also includes a cover for covering and enclosing the tissue and the tissue growth medium. The cover also functions to cover and enclose the open-cell foam section, when used. In applications where the cover is not self-sealing by suction, the appliance may also include sealing means for sealing the cover to the region surrounding the tissue to be grown in order to maintain reduced pressure in the vicinity of the tissue during tissue growth. When the cover is sealed in position over the tissue site, a generally fluid-tight or gas-tight sealed enclosure is formed over or about the tissue site. The sealing means may be in the form of an adhesive applied to the underside of the cover or at least to the periphery of the underside of the cover for sealing the cover to the region surrounding the tissue. The sealing means may also include a separate sealing member, such as an adhesive strip, a sealing ring, a tubular pad or an inflatable cuff, for use with the cover for positioning around the region surrounding the tissue to be grown. In selected embodiments, the reduced pressure within the sealed enclosure beneath the cover may serve to self adhere and seal the cover in position at the tissue growth site. The reduced pressure appliance may also include a suction port for enabling reduced pressure to be supplied within the sealed volume enclosed beneath the cover. The suction port may be in the form of a nipple on the cover. Alternatively, the suction port may be in the form of a tube attached to the cover. As yet another alternative, the port may be provided at the mouth of a suction tube that is inserted beneath the cover.

A vacuum system is connected with the reduced pressure appliance in order to provide suction or reduced pressure to the appliance. For this purpose, the vacuum system includes a suction pump or suction device for connection with the suction port of the appliance for producing the reduced pressure over the tissue site. The vacuum system may include a section of hose or tube, such as a vacuum hose, that interconnects the suction device with the suction port of the appliance to provide the reduced pressure at the tissue site. A tube of the appliance may serve as the vacuum hose for connection with the suction device. Further, a mouth of the suction hose may serve as the suction port in applications where the suction hose is not connected to a separate port.

A collection device in the form of a fluid trap may be provided intermediate the vacuum hose of the suction device and the suction port of the appliance to trap any exudate which may be aspirated from the tissue by the reduced pressure appliance. A stop mechanism may also be provided for the vacuum system to halt application of the reduced pressure at the tissue site in the event that a predetermined quantity of exudate has been collected. The apparatus may also include a control device for controlling the pump and for providing intermittent or cyclic production of reduced pressure.

In a particular embodiment of the invention, the cover for the reduced pressure appliance may be in the form of a gas impermeable covering sheet of flexible polymer material, such as polyurethane, having an adhesive backing that provides the seal for securing the sheet over the tissue site to provide a gas-tight or fluid-tight sealed enclosure over the tissue site. A semi-permeable cover may also be used in selected applications. The vacuum system of the tissue treatment apparatus may include a suction pump having a vacuum hose that is connected with or, alternatively, integral with, a suction tube serving as a suction port for the appliance. The suction tube for the appliance runs beneath the cover sheet when sealed in position over the tissue site and into the fluid-tight enclosure provided at the tissue site beneath the cover sheet. An adhesive backing on the cover sheet is used to provide a fluid-tight seal around the feedthrough for the suction tube at the tissue site. Within the enclosure, the suction tube is connected to a section of open-cell foam in communication with the tissue growth medium into which the tissue may be grown. The tissue growth medium may take the form of a layer of skin-substitute material, for example. The open-cell foam functions to more uniformly apply reduced pressure or suction over the tissue growth medium. Likewise, the open cell foam section communicates the reduced pressure to the tissue site under the cover sheet while holding the cover sheet substantially out of contact with the tissue during the application of reduced pressure at the enclosed tissue site. Alternatively, the tissue growth medium may comprise a porous structure, such as a bone substitute material. In such a case, the suction tube may be connected directly with the tissue growth medium, and the open-cell foam section may be omitted.

A method of treating tissue damage is also provided which comprises applying a reduced pressure to a tissue to be grown over an area sufficient to promote the growth of new cells within a tissue growth medium and/or the tissue. The method of growing a tissue comprises providing a tissue growth medium proximate the tissue to be grown. A reduced pressure is subsequently applied to the tissue growth medium and the tissue. The reduced pressure is maintained until the tissue has progressed toward a selected stage of growth in the medium. The reduced pressure may be provided in alternating periods of application and non-application of the reduced pressure.

More specifically, the application of reduced pressure comprises the steps of placing a tissue growth medium in contact with a tissue to be grown; locating a cover over the tissue; sealing the cover about the tissue to form a reduced pressure chamber; and operably connecting the cover or at least the reduced pressure chamber with a vacuum system for producing the reduced pressure. In specific application, the method includes maintaining the reduced pressure until the tissue has progressed toward a selected stage of growth within the tissue growth medium.

### Brief Description of the Drawings

The foregoing summary, as well as the following detailed description of the preferred embodiments of the present invention, will be better understood when read in conjunction with the appended drawings, in which:

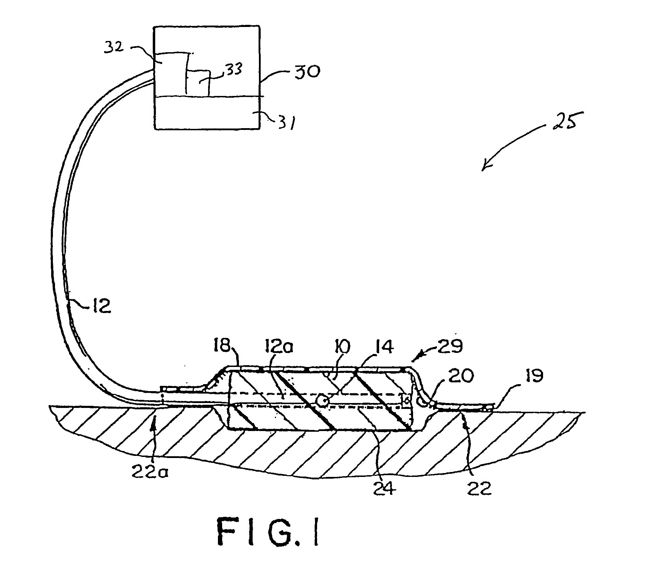

Figure 1 is a schematic cross-sectional view of a reduced pressure appliance comprising a porous tissue growth medium, a flexible hose for connecting the tissue growth medium with a vacuum system, and an adhesive-backed flexible polymer sheet overlying the growth medium to provide a seal over a tissue to be grown; and

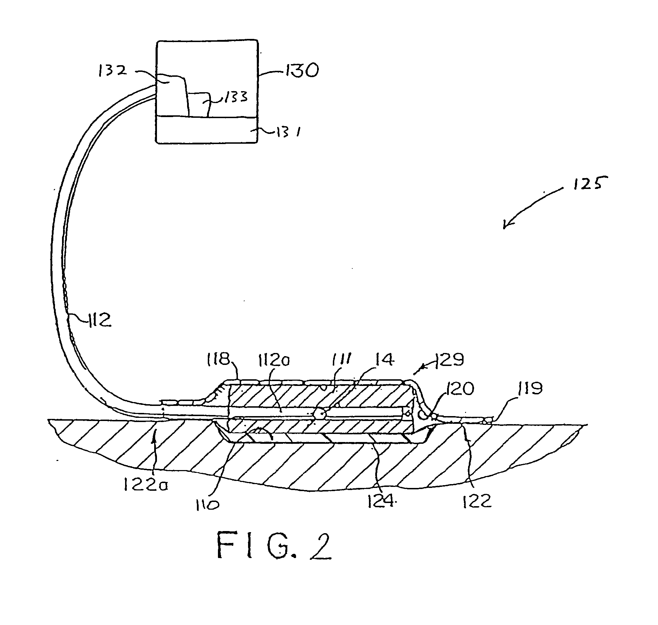

Figure 2 is a schematic cross-sectional view of a reduced pressure appliance comprising a foam section in communication with a tissue growth medium, a flexible hose for connecting the foam section with a vacuum system, and an adhesive-backed flexible polymer sheet overlying the growth medium-foam section assembly to provide a seal over a tissue to be grown.

### Detailed Description of the Preferred Embodiments

In accordance with the present invention, a directed tissue growth apparatus is provided for promoting growth in a tissue by application of reduced pressure (i.e., below atmospheric pressure) so that suction may be applied to a tissue site in a controlled manner for a selected time period.

Referring to Fig. 1, a directed tissue growth apparatus, generally designated 25, is depicted having a reduced pressure appliance 29 for enclosing a tissue site to provide a fluid-tight or gas-tight enclosure over the tissue site to grow tissue 24 in a tissue growth medium 10 through the application of sub-atmospheric pressure. The directed tissue growth apparatus 25 includes a reduced pressure appliance, generally designated 29, which is applied to and sealed over a tissue site in order to enclose the tissue site to form a reduced pressure chamber about the tissue site for treatment with suction or reduced pressure within a sealed generally fluid-tight or gas-tight enclosure. For the purpose of creating suction within the appliance 29, the appliance 29 is connected with a vacuum system, generally designated 30, to provide a source of suction or reduced pressure for the sealed appliance 29 at the tissue site. The vacuum system 30 may include a suction device 31 and an optional fluid collection device 32 intermediate the hose 12 and suction device 31. The fluid collection device 32 functions to collect any exudate that may be aspirated from the tissue. A stop mechanism 33 may be provided to halt application of the suction device 31 upon collection of a predetermined quantity of fluid in the fluid collection device 32. The appliance 29 includes a fluid-impermeable tissue cover 18 in the form of a flexible, adhesive, fluid impermeable polymer sheet for covering and enclosing the tissue 24 at the tissue site. The tissue cover 18 includes an adhesive backing 20 which functions to seal the tissue cover about the periphery of the tissue 24 to provide a generally gas-tight or fluid-tight enclosure over the tissue 24. The adhesive cover sheet 18 must have sufficient adhesion to form a fluid-tight or gas-tight seal 19 around the tissue 24 and to hold the sheet 18 in sealed contact at the attachment site during the application of suction or reduced pressure. Alternatively, the cover 18 may be provided in the form of a rigid or semi-rigid cover adapted to form a fluid-tight or gas-tight seal around the tissue. Suitable modifications may be made to the appliance to provide a reduced pressure chamber for treating the selected tissue.

The appliance 29 also includes a porous tissue growth medium 10 which is positioned under the cover 18 on or in the tissue 24. The tissue growth medium 10 is disposed over a sufficient expanse of the tissue 24 to promote sufficient growth of new tissue cells within the tissue growth medium 10. The tissue growth medium 10 should be sufficiently porous to allow for connection to a vacuum system 30 to supply sub-atmospheric pressure to the tissue 24 and the tissue growth medium 10. The tissue growth medium 10 may also be perforated to enhance gas flow and to reduce the weight of the appliance. The configuration and composition of the tissue growth medium 10 can be adjusted to suit the particular tissue type. For example, for growth in bone tissue, the tissue growth medium 10 may comprise a natural, synthetic, or natural-synthetic hybrid porous material. Such a tissue growth medium 10 may conveniently be chosen to provide a scaffold to support or direct osteoconduction, i.e. bone formation. Alternatively or additionally, such a tissue growth medium 10 may be selected from materials which induce differentiation of stem cells to osteogenic cells, i.e. osteoinductive agents, or materials which provide stem cells, e.g. bone marrow aspirate.

For example, a tissue growth medium 10 for use in bone growth may be a bioglass, ceramic material, or other natural or synthetic porous material. In particular, materials comprising calcium sulphate or calcium phosphate are suited for use as a bone tissue growth medium 10. A calcium sulfate bone substitute is completely absorbed by osteoclasts, and osteoblasts will attach to the calcium sulfate bone substitute and lay osteoid on it. The process of absorption by osteoclasts is complete and quick. Hence, it can be used with antibiotics in presence of infection. A chief advantage is that a calcium sulfate bone substitute can be used in presence of infection as well as being one of the least expensive bone substitutes. Calcium sulphate bone substitutes principally possess osteoconductive properties and no osteoinductive properties, though such a material could be modified to provide osteoinductive properties. One suitable calcium sulphate bone substitute is OSTEOSET® Bone Graft Substitute, a product of Wright Medical Technology, Inc. of Arlington TN.

Another class of suitable materials is one comprising various derivates of calcium phosphate, which can be used to provide a structural matrix for osteoconduction. These derivatives also do not possess any osteoinductive properties. The commonly used derivates are: hydroxyapatite (coral based or chemically derived synthetic ceramic), fluorapatite, tri-calcium phosphate, bioglass ceramics and combinations thereof. One suitable calcium phosphate bone substitute is OsteoGraft™ Bone Graft Substitute, a product of Millenium Biologix of Kingston, Ontario, Canada.

The appliance 29 also includes a suction port in the form of a hollow suction tube 12 that connects either directly or indirectly with the vacuum system 30 to provide suction within the sealed enclosure. The outlet of suction tubing 12 serves as a suction port for the appliance 29. An end segment 12a of the tubing 12 is embedded within the tissue growth medium 10 for providing suction or reduced pressure within the chamber provided under the tissue cover 18. The open-cell structure of the tissue growth medium 10 also permits the tissue growth medium 10 to distribute the reduced pressure within the chamber. Embedding the open end of segment 12a of tubing 12 within the interior of the tissue growth medium 10 permits the tissue growth medium 10 to function as a shield to help prevent the tissue cover 18 from being inadvertently sucked into occlusive engagement with the open end of the tube thereby plugging the tube 12 and restricting gas flow. The open-cell structure of the tissue growth medium 10 also permits the tissue growth medium 10 to distribute the reduced pressure within the sealed enclosure.

The tube segment 12a embedded within the tissue growth medium 10 optionally has at least one side port 14 for positioning within the interior of the tissue growth medium 10 to promote substantially uniform application of reduced pressure throughout the enclosure. Positioning the side port 14 of tube segment 12a within the interior of the tissue growth medium 10 permits the tissue growth medium 10 to also function as a shield for the side port to thereby prevent the tissue cover 18 from being sucked into the side port 14 and thereby restricting gas flow. The open cells of the tissue growth medium 10 facilitate growth of tissue therein and facilitate gas flow throughout the enclosure. In addition, the tissue growth medium 10 functions to hold the tissue cover 18 generally out of contact with the tissue 24 during the application of suction within the enclosure.

Tubing 12 and tube segment 12a may be sufficiently flexible to permit movement of the tubing but are sufficiently rigid to resist constriction when reduced pressure is supplied to the appliance 29 or when the location of the tissue is such that the patient must sit or lie upon the tubing 12 or upon the reduced pressure appliance 29. The assembly comprising the tissue growth medium 10 and the tube 12 may be fabricated by snaking the end of the tube segment 12a through an internal passageway in the tissue growth medium 10 such as by pulling the end of the tube segment 12a through the passageway using forceps. The assembly is preferably prepared prior to use under sterile conditions and then stored in an aseptic package.

In order to use the reduced pressure appliance 29 at the site of the tissue 24, the flexible, fluid-impermeable, adhesive tissue cover sheet 18 is secured in position at the tissue site, overlying the tissue growth medium 10 disposed in contact with the tissue 24. The tissue cover sheet 18 is secured and sealed to the attachment site 22 by an adhesive layer 20 on the under surface of the tissue cover 18 to form a gas-tight seal 19 about the tissue 24. The tissue cover 18 also provides a gas-tight seal around the tubing 12 at the feedthrough location 22a where the tubing 12 emerges from beneath the tissue cover 18. The tissue cover 18 is preferably formed of a fluid impermeable or gas impermeable flexible adhesive sheet such as Ioban, a product of the 3M Corporation of Minneapolis, MN.

Predetermined amounts of suction or reduced pressure may be produced by the vacuum system 30. The vacuum system 30 is preferably controlled by a control device or control circuitry that includes a switch or a timer which may be set to provide cyclic on/off operation of the vacuum system 30 according to user-selected intervals. Alternatively, the vacuum system 30 may be operated continuously without the use of a cyclical timer. The control may also include a pressure selector to enable the amount of suction produced by the system to be adjusted so that a suitable sub-atmospheric pressure may be created within the chamber. Operation of the vacuum system 30 may be controlled to permit graduated increases in the amount of vacuum applied or graduated decreases in the amount of vacuum applied.

Referring to Fig. 2, an alternative configuration of the reduced pressure appliance 129 is shown which is similar to the reduced pressure appliance 29 of Fig. 1. The elements of the appliance 129 of Fig. 2 which are similar to like elements depicted in Fig. 1 utilize the same reference number as used in Fig. 1 but with a 100-series added to such reference numerals. A principal difference between the reduced pressure appliance 129 of Fig. 2 and that of Fig. 1 is that a porous open-cell foam section 111 is provided for communication with the vacuum system 130. The open-cell foam section 111 in turn communicates with the tissue growth medium 110, which may or may not be porous.

The directed tissue growth apparatus 125 includes a reduced pressure appliance, generally designated 129, which is applied to and sealed over a tissue site in order to enclose the tissue site for treatment with suction or reduced pressure within a sealed generally fluid-tight or gas-tight enclosure. For the purpose of creating suction within the appliance 129, the appliance 129 is connected with a vacuum system, generally designated 130, to provide a source of suction or reduced pressure for the sealed appliance 129 at the tissue site. The appliance 129 includes a fluid-impermeable tissue cover 118 in the form of a flexible, fluid impermeable polymer sheet for covering and enclosing the tissue 124 at the tissue site. The tissue cover 118 may include an adhesive backing 120 at least around the periphery of the cover which functions to seal the tissue cover proximate to the tissue 124 to provide a generally gas-tight or fluid-tight enclosure over the tissue 124. The adhesive cover sheet 118 must have sufficient adhesion to form a fluid-tight or gas-tight seal 119 around the tissue 124 and to hold the sheet 118 in sealed contact with the attachment site around the tissue 124 during the application of suction or reduced pressure. The cover 118 may also be provided in the form of a rigid or semi-rigid cover which cooperates with a suitable seal to form a fluid-tight or gas-tight seal around the tissue.

The appliance 129 also includes a porous open-cell foam section 111 which is placed under the cover 118 and in direct or indirect contact with a tissue growth medium 110. The open-cell foam section 111 should be sufficiently porous to allow for connection to the vacuum system 130 to transmit sub-atmospheric pressure to the tissue 124 and the tissue growth medium 110. The tissue growth medium 110 is placed over a sufficient expanse of the tissue 124 to promote sufficient growth of new tissue cells within the tissue growth medium 110. The tissue growth medium 110 and/or the foam section 111 may also be perforated or channeled to enhance gas flow and to reduce the weight of the appliance. The configuration depicted in Fig. 2 is particularly suitable for use with non-porous growth media or a relatively thin growth medium, such as a skin substitute material. A skin substitute material may comprise a multilayer structure having, for example, a layer of collagen for contact with the tissue to be grown and a silicone layer disposed on top of the collagen layer for contact with the foam section 111. A suitable skin substitute material is INTEGRA^{®} Dermal Regeneration Template, a product of Integra LifeSciences Corp. of Plainsboro, NJ. The silicone layer provides a removable backing to support the collagen layer during tissue ingrowth. After growth has continued to a desired stage, the silicone layer may be removed from the collagen layer, leaving the neodermis in place, onto which a thin split thickness skin graft may be placed.

For another application, the growth medium 110 can be formed for the purpose of growing an organ, such as the pancreas or liver. The growth medium 110 may take the form of a scaffold/matrix of either a naturally occurring molecule (e.g., collagen) or of resorbable materials (e.g., polyglycolic acid or polygalactic acid or a combination thereof). The growth medium 110 may be formed from commercially available screens which are layered to the desired thickness.

The appliance 129 also includes a suction port in the form of a hollow suction tube 112, similar to the tube 12 of Fig. 1, that connects with the vacuum system 130 to provide suction within the sealed enclosure. The suction tubing 112 provides at least one suction port for the appliance 129. Unlike the device of Fig. 1, an end segment 112a of the tubing 112 is embedded within the foam section 111, rather than in the tissue growth material, for providing suction or reduced pressure within the enclosure provided under the tissue cover 118. The open-cell structure of the foam section 111 permits the foam section 111 to distribute the reduced pressure within the enclosure. Embedding the open end of segment 112a of tubing 112 within the interior of the foam section 111 permits the foam section 111 to function as a shield to help prevent the tissue cover 118 from being inadvertently sucked into the open end of the tube thereby plugging the tube 112 and restricting gas flow. The open-cell structure of the foam section 111 also permits the foam section 111 to distribute the pressure within the sealed enclosure.

The tube segment 112a embedded within the foam section 111 preferably has at least one side port 114 for positioning within the interior of the foam section 111 to promote substantially uniform application of reduced pressure throughout the enclosure. Positioning the side port 114 of tube segment 112a within the interior of the foam section 111 permits the foam section 111 to function as a shield for the side port to thereby prevent the tissue cover 118 from being sucked into the side port 114 and thereby restricting gas flow. The open cells of the foam section 111 facilitate gas flow throughout the enclosure. In addition, the foam section 111 functions to hold the tissue cover 118 generally out of contact with the tissue 124 during the application of suction within the enclosure.

Similar to the appliance 29 of Fig. 1, the flexible, fluid-impermeable, adhesive tissue cover sheet 118 of appliance 129 is secured, during use, in position at the tissue site, overlying the foam section 111 and tissue growth medium 110 which contacts the tissue 124. The tissue cover sheet 118 is secured and sealed to the attachment site 122 by an adhesive layer 120 on the under surface of the tissue cover 118 to form a gas-tight seal 119 about the tissue 124. The tissue cover 118 also provides a gas-tight seal around the tubing 112 at the feedthrough location 122a where the tubing 112 emerges from beneath the tissue cover 118.

Reduced pressure appliances are useful for growing a variety of tissues. Directed growth of a tissue can be carried out by securing a reduced pressure appliance to the treatment site as previously shown and described, and then maintaining a substantially continuous or cyclical reduced pressure within the appliance until the newly grown tissue has reached a desired degree of development. The method may be practiced using a subatmospheric pressure ranging from about 0.5 to about 0.98 atmospheres, and more specifically about .73 atmospheres to about .95 atmospheres, and more preferably practiced using a subatmospheric pressure ranging between about 0.8 to about 0.9 atmospheres. The time period for use of the method on a tissue may preferably be at least 48 hours, but can, for example, be extended for multiple days. Satisfactory growth of various types of tissues has been obtained via the use of reduced pressures equivalent to about 1 to 8 in. Hg below atmospheric pressure.

Supplying reduced pressure to the appliance in an intermittent or cyclic manner may be desirable for growing tissues. Intermittent or cyclic supply of reduced pressure to an appliance may be achieved by manual or automatic control of the vacuum system. A cycle ratio, the ratio of "on" time to "off" time, in such an intermittent reduced pressure treatment may be as low as 1:10 or as high as 10:1. The preferred ratio is approximately 5 minutes on which is usually accomplished in alternating intervals of 5 minutes of reduced pressure supply and 2 minutes of non-supply.

A suitable vacuum system includes any suction pump capable of providing at least 5 mm of Hg of suction to the tissue, and preferably up to 125 mm of Hg suction, and most preferably up to approximately 200 mm of Hg of suction or even higher as necessary to achieve subatmospheric pressures of about .5 atmospheres. The pump can be any ordinary suction pump suitable for medical purposes that is capable of providing the necessary suction. The dimension of the tubing interconnecting the pump and the reduced pressure appliance is controlled by the pump's ability to provide the suction level needed for operation. For example, a 1/4 inch diameter tube may be suitable.

The present invention also includes a method of growing tissue which comprises the steps of applying reduced pressure to a tissue for a selected time and at a selected magnitude sufficient to promote cell growth in a matrix or scaffold material. Further features of the apparatus and method for use thereof shall be made apparent in the following example.

### Example 1

This example was designed to demonstrate the effectiveness of the method of the invention for accelerating the rate of vascular ingrowth in a skin substitute material.

Several 25 kg pigs were sedated and prepped for surgery. A series of 2 cm by 5 cm full thickness defects (down to the deep back muscles) were created on each side of the back of the animal. A directed tissue growth apparatus of the type shown in Fig. 2 comprising INTEGRA^{®} Dermal Regeneration Template was applied to the wound site on one side of each animal. An INTEGRA^{®} Dermal Regeneration Template was applied to the wound site on the other side of each animal. The INTEGRA^{®} Dermal Regeneration Template was applied according the manufacturer's directions. Vacuum (125 mm Hg, continuous) was applied to the directed tissue growth apparatus positioned on the selected wound sites on one side of the pigs. After 72 hours, the apparatus was removed, and the pieces of INTEGRA^{®} Dermal Regeneration Template were peeled off from the vacuum-treated and non-vacuum-treated wound sites at 10 cm/sec. The force required to pull the pieces off was recorded. A paired T-test was used to determine statistical significance of the pull force data.

The INTEGRA^{®} Dermal Regeneration Template at the non-vacuum-treated wound site required a force of 1.25 x 10⁵ +/- 0.51 x 10⁵ dynes to separate the template from the wound site. The INTEGRA^{®} Dermal Regeneration Template at the vacuum-treated wound site required a greater force of 2.25 x 10⁵ +/- 0.51 x 10⁵ dynes to separate the vacuum-treated template from the wound site. The increased pull force required at the vacuum-treated wound site indicated increased ingrowth of vasculature in the skin substitute material due to the vacuum treatment. The increased vascular ingrowth was confirmed by micrographs of the vacuum-treated and non-vacuum-treated pieces of INTEGRA^{®} Dermal Regeneration Template removed from the animals.

The terms and expressions which have been employed are used as terms of description and not of limitation and there is no intention in the use of such terms and expressions of excluding any equivalents of the features shown and described, or portions thereof, but it is recognized that various modifications are possible within the scope of the claimed invention.

Clauses relating to the invention and forming part of the description
1. An appliance for administering a reduced pressure treatment to promote directed growth of a tissue comprising:
   (a) a cover adapted to cover and enclose a tissue to be grown and adapted to maintain reduced pressure at the site of the tissue;
   (b) a seal adapted to seal the cover about the tissue;
   (c) reduced pressure supply means for connection to a source of suction, the reduced pressure supply means cooperating with the cover to supply the reduced pressure beneath the cover; and
   (d) a bone substitute material adapted to promote tissue growth therein, the bone substitute material being located between the tissue and the cover.
2. An appliance according to clause 1 wherein the bone substitute material comprises a bioabsorbable material.
3. An appliance according to clause 1 wherein the bone substitute material comprises at least one of calcium sulfate, calcium phosphate, a bioglass, or a ceramic.
4. An appliance according to clause 1 wherein the cover comprises a flexible sheet.
5. An appliance according to clause 1 wherein the bone substitute material comprises a porous material.
6. An appliance according to clause 1 wherein the seal includes an adhesive material on the cover adapted to secure the cover to the tissue to be grown.
7. An appliance according to clause 1 wherein the reduced pressure supply means includes a segment of tubing embedded within the bone substitute material.
8. An apparatus for growing a tissue, comprising:
   vacuum means for creating a sub-atmospheric pressure at a tissue to be grown;
   sealing means operatively associated with the vacuum means for maintaining the sub-atmospheric pressure at the tissue by contacting the region surrounding the tissue; and
   a bone substitute material for positioning at the tissue within the sealing means and adapted to promote tissue growth therein.
9. An apparatus according to clause 8 wherein the bone substitute material comprises a bioabsorbable material.
10. An apparatus according to clause 8 wherein the bone substitute material comprises at least one of calcium sulfate, calcium phosphate, a bioglass, or a ceramic.
11. An apparatus according to clause 8, wherein the sealing means includes a flexible sealing rim in contact with the region surrounding the tissue.
12. An apparatus according to clause 8, wherein the sealing means includes a flexible polymer sheet overlying the bone substitute material, the polymer sheet having adhesive on at least a surface facing the tissue to attach and seal the polymer sheet to the region surrounding the tissue.
13. An apparatus according to clause8, wherein the sealing means comprises a fluid-impermeable cover.
14. An apparatus according to clause 8 wherein the vacuum means supplies a sub-atmospheric pressure between about 0.5 and 0.98 atmospheres to the tissue.
15. An apparatus according to clause 8 wherein the vacuum means supplies a sub-atmospheric pressure between about 0.73 and 0.95 atmospheres to the tissue.
16. An apparatus according to clause 8 wherein the vacuum means supplies a sub-atmospheric pressure between about 0.8 and 0.9 atmospheres to the tissue.
17. An apparatus according to clause 8, wherein the vacuum means operates continuously.
18. An apparatus according to clause 8, wherein the vacuum means operates cyclically to provide periods of application and non-application of suction.
19. An apparatus according to clause 18 wherein the vacuum means provides periods of application and non-application of suction with the ratio of duration of application period to non-application period between about 1:10 and 10:1.
20. An apparatus according to clause 19 wherein the duration of the application period is about 5 minutes.
21. An apparatus according to clause 20 wherein the duration of the non-application period is about 2 minutes.
22. An apparatus for applying sub-atmospheric pressure to a tissue to be grown beneath a fluid-impermeable seal comprising:
   a bone substitute material for positioning beneath the seal configured to overlie the tissue such that the sub-atmospheric pressure is maintained within the bone substitute material and is applied to the tissue; and
   a flexible tube having an inlet end inserted into the bone substitute material and an outlet end for extending from beneath the seal for supplying the subatmospheric pressure.
23. An apparatus according to clause 22 wherein the bone substitute material comprises a bioabsorbable material.
24. An apparatus according to clause22 wherein the bone substitute material comprises at least one of calcium sulfate, calcium phosphate, a bioglass, or a ceramic.
25. An apparatus for growing a tissue, comprising:
   a bone substitute material configured to overlie a tissue to be grown;
   a fluid-impermeable cover overlying the bone substitute material, the cover adapted to form a seal with the region surrounding the tissue for maintaining sub-atmospheric pressure beneath the cover; and
   a tubular member having a first end inserted within a portion of the bone substitute material and having a second end extending from beneath the cover to a location external to the cover for supplying sub-atmospheric pressure beneath the cover.
26. An apparatus according to clause 25 wherein the bone substitute material comprises a bioabsorbable material.
27. An apparatus according to clause25 wherein the bone substitute material comprises at least one of calcium sulfate, calcium phosphate, a bioglass, or a ceramic.
28. An apparatus according to clause 25 wherein the first end of the tubular member is embedded within the bone substitute material.
29. An apparatus for growing a tissue, comprising:
   vacuum means for creating a sub-atmospheric pressure at a tissue to be grown;
   sealing means operatively associated with the vacuum means for maintaining the sub-atmospheric pressure at the tissue by contacting the region surrounding the tissue; and
   a bone substitute material for positioning at the tissue within the sealing means, the bone substitute material having a pore size sufficiently large to permit tissue growth therein.
30. An apparatus according to clause29, wherein the bone substitute material comprises a bioabsorbable material.
31. An apparatus according to clause 29, wherein the bone substitute material comprises at least one of calcium sulfate, calcium phosphate, a bioglass, or a ceramic.
32. An apparatus according to clause 29, wherein the sealing means includes a flexible sealing rim in contact with the region surrounding the tissue.
33. An apparatus according to clause 29, wherein the sealing means includes a flexible polymer sheet overlying the bone substitute material, the polymer sheet having adhesive on at least a surface facing the tissue to attach and seal the polymer sheet to the region surrounding the tissue.
34. An apparatus according to clause 29, wherein the vacuum means operates continuously.
35. An apparatus according to clause 29, wherein the vacuum means operates cyclically to provide periods of application and non-application of suction.
36. An apparatus according to clause 35, wherein the vacuum means operates cyclically to provide periods of application and non-application of suction with the ratio of duration of application period to non-application period between about 1:10 and 10:1.
37. An apparatus according to clause 36 wherein the duration of the application period is about 5 minutes.
38. An apparatus according to clause 37 wherein the duration of the non-application period is about 2 minutes.
39. An apparatus for growing a tissue comprising:
   a bone substitute material configured to overlie the tissue;
   a cover overlying the bone substitute material, the cover adapted to form a seal with the region surrounding the tissue for maintaining a sub-atmospheric pressure beneath the cover;
   a tubular member having a first end inserted within a portion of the bone substitute material and having a second end extending from beneath the cover to a location external to the cover; and
   a vacuum source connected with the second end of the tubular member for supplying the sub-atmospheric pressure to the tissue.
40. An apparatus according to clause 39 wherein the bone substitute material comprises a bioabsorbable material.
41. An apparatus according to clause 39 wherein the bone substitute material comprises at least one of calcium sulfate, calcium phosphate, a bioglass, or a ceramic.
42. An apparatus according to clause 39 wherein the vacuum source supplies a sub-atmospheric pressure between about 0.5 and 0.98 atmospheres to the tissue.
43. An apparatus according to clause 39 wherein the vacuum source supplies a sub-atmospheric pressure between about 0.73 and 0.95 atmospheres to the tissue.
44. An apparatus according to clause 39 wherein the vacuum source supplies a sub-atmospheric pressure between about 0.8 and 0.9 atmospheres to the tissue.
45. An apparatus according to clause 39 wherein the first end of the tubular member is embedded within the bone substitute material.
46. An apparatus for growing a tissue comprising:
   a bioabsorbable scaffold material configured to overlie the tissue, the scaffold material having a pore size sufficiently large to permit tissue growth therein;
   an open-cell foam section in communication with the scaffold material;
   a cover overlying the scaffold material and the foam section, the cover adapted to form a seal with the region surrounding the tissue for maintaining a sub-atmospheric pressure beneath the cover; and
   a tubular member having a first end inserted within a portion of the foam section and having a second end extending from beneath the cover to a location external to the cover for supplying the sub-atmospheric pressure beneath the cover.
47. An apparatus according to clause 46 wherein the scaffold material comprises a skin substitute material.
48. An apparatus according to clause 46 wherein the scaffold material comprises a collagen layer.
49. An appliance for administering a reduced pressure treatment to promote directed growth of a tissue comprising:
   (a) a cover adapted to cover and enclose a tissue to be grown and adapted to maintain reduced pressure at the site of the tissue;
   (b) a seal adapted to seal the cover about the tissue;
   (c) reduced pressure supply means for connection to a source of suction, the reduced pressure supply means cooperating with the cover to supply the reduced pressure beneath the cover; and
   (d) a bioabsorbable scaffold material for promoting tissue growth therein configured to be located between the tissue and the cover.
50. An appliance according to clause 49 wherein the scaffold material comprises a skin substitute material.
51. An appliance according to clause 49 wherein the scaffold material comprises a collagen layer.
52. An appliance according to clause 49 wherein the cover comprises a flexible sheet.
53. An appliance according to clause 49 wherein the seal includes an adhesive material on the cover adapted to secure the cover to the tissue to be grown.
54. An appliance according to clause 49 comprising an open-cell foam section in communication with the scaffold material.
55. An appliance according in clause 54 wherein the reduced pressure supply means includes a segment of tubing embedded within the foam section.
56. An apparatus for growing a tissue, comprising:
   vacuum means for creating a sub-atmospheric pressure at a tissue to be grown;
   sealing means operatively associated with the vacuum means for maintaining the sub-atmospheric pressure at the tissue by contacting the region surrounding the tissue; and
   a bioabsorbable scaffold material for positioning at the tissue within the sealing means and adapted to promote tissue growth therein.
57. An apparatus according to clause 56 wherein the scaffold material comprises a skin substitute material.
58. An apparatus according to clause 56 wherein the scaffold material comprises a collagen layer.
59. An apparatus according to clause 56, wherein the sealing means includes a flexible sealing rim in contact with the region surrounding the tissue.
60. An apparatus according to clause 56, wherein the sealing means comprises a fluid-impermeable cover.
61. An apparatus according to clause 56, wherein the sealing means includes a flexible polymer sheet overlying the scaffold material, the polymer sheet having adhesive on at least a surface facing the tissue to attach and seal the polymer sheet to the region surrounding the tissue.
62. An apparatus according to clause 56 comprising an open-cell foam section in communication with the scaffold material.
63. An apparatus according to clause 62 wherein the vacuum means includes a segment of tubing embedded within the foam section.
64. An apparatus according to clause 56, wherein the vacuum means operates continuously.
65. An apparatus according to clause 56, wherein the vacuum means operates cyclically to provide periods of application and non-application of suction.
66. An apparatus according to clause 65 wherein the vacuum means provides periods of application and non-application of suction with the ratio of duration of application period to non-application period between about 1:10 and 10:1.
67. An apparatus according to clause 66 wherein the duration of the application period is about 5 minutes.
68. An apparatus according to clause 67 wherein the duration of the non-application period is about 2 minutes.
69. An apparatus according to clause 56 wherein the vacuum means supplies a sub-atmospheric pressure between about 0.5 and 0.98 atmospheres to the tissue.
70. An apparatus according to clause 56 wherein the vacuum means supplies a sub-atmospheric pressure between about 0.73 and 0.95 atmospheres to the tissue.
71. An apparatus according to clause 56 wherein the vacuum means supplies a sub-atmospheric pressure between about 0.8 and 0.9 atmospheres to the tissue.
72. An apparatus for applying sub-atmospheric pressure to a tissue to be grown beneath a fluid-impermeable seal comprising:
   a bioabsorbable scaffold material for positioning beneath the seal configured to overlie the tissue; and
   a flexible tube having an inlet end inserted into the scaffold material and an outlet end for extending from beneath the seal for supplying a sub-atmospheric pressure to the tissue.
73. An apparatus according to clause 72 wherein the scaffold material has a pore size sufficiently large to permit tissue growth therein.
74. An apparatus according to clause 72 wherein the scaffold material comprises a skin substitute material.
75. An apparatus according to clause 72 wherein the scaffold material comprises collagen.
76. An apparatus for growing a tissue, comprising:
   a bioabsorbable scaffold material configured to overlie a tissue to be grown;
   an open-cell foam section in communication with the scaffold material;
   a fluid-impermeable cover overlying the scaffold material and the foam section, the cover adapted to form a seal with the region surrounding the tissue for maintaining sub-atmospheric pressure beneath the cover; and
   a tubular member having a first end inserted within a portion of the foam section and having a second end extending from beneath the cover to a location external to the cover for supplying sub-atmospheric pressure beneath the cover.
77. An apparatus according to clause 76 wherein the scaffold material comprises a skin substitute material.
78. An apparatus according to clause 77 wherein the scaffold material comprises a collagen layer.
79. An apparatus for growing a tissue, comprising:
   vacuum means for creating a sub-atmospheric pressure at a tissue to be grown;
   sealing means operatively associated with the vacuum means for maintaining the sub-atmospheric pressure at the tissue by contacting the region surrounding the tissue; and
   a bioabsorbable scaffold material for positioning at the tissue within the sealing means, the scaffold material having a pore size sufficiently large to permit tissue growth therein.
80. An apparatus according to clause 79, wherein the scaffold material comprises a skin substitute material.
81. An apparatus according to clause 79, wherein the scaffold material comprises collagen.
82. An apparatus according to clause 79, wherein the sealing means includes a flexible sealing rim in contact with the region surrounding the tissue.
83. An apparatus according to clause 79, wherein the cover includes a flexible polymer sheet overlying the scaffold material, the polymer sheet having adhesive on at least a surface facing the tissue to attach and seal the polymer sheet to the region surrounding the tissue.
84. An apparatus according to clause 79 wherein the vacuum means includes a segment of tubing embedded within the scaffold material.
85. An apparatus according to clause 79, wherein the vacuum means operates continuously.
86. An apparatus according to clause 79, wherein the vacuum means operates cyclically to provide periods of application and non-application of suction.
87. An apparatus according to clause 86, wherein the vacuum means operates cyclically to provide periods of application and non-application of suction with the ratio of duration of application period to non-application period between about 1:10 and 10:1.
88. An apparatus according to clause 87 wherein the duration of the application period is about 5 minutes.
89. An apparatus according to clause 88 wherein the duration of the non-application period is about 2 minutes.
90. An apparatus for growing a tissue comprising:
   a bioabsorbable scaffold material configured to overlie the tissue;
   a cover overlying the scaffold material, the cover adapted to form a seal with the region surrounding the tissue for maintaining a sub-atmospheric pressure beneath the cover;
   a tubular member having a first end inserted within a portion of the scaffold material and having a second end extending from beneath the cover to a location external to the cover; and
   a vacuum source connected with the second end of the tubular member for supplying the sub-atmospheric pressure to the tissue.
91. An apparatus according to clause 90 wherein the scaffold material comprises a skin substitute material.
92. An apparatus according to clause 90 wherein the scaffold material comprises a collagen layer.
93. An apparatus according to clause 90 wherein the vacuum source supplies a sub-atmospheric pressure between about 0.5 and 0.98 atmospheres to the tissue.
94. An apparatus according to clause 90 wherein the vacuum source supplies a sub-atmospheric pressure between about 0.73 and 0.95 atmospheres to the tissue.
95. An apparatus according to clause 90 wherein the vacuum source supplies a sub-atmospheric pressure between about 0.8 and 0.9 atmospheres to the tissue.
96. A method of growing a tissue comprising the steps of:
   (a) providing a tissue growth medium proximate the tissue;
   (b) applying a sub-atmospheric pressure to a tissue to be grown; and
   (c) maintaining the sub-atmospheric pressure until the tissue has progressed toward a selected stage of growth within the tissue growth medium.
97. The method according to clause 96, wherein the maintaining of the sub-atmospheric pressure is conducted in alternating periods of application and non-application of the sub-atmospheric pressure.
98. The method according to clause 97, wherein each of the alternating periods is about 5 minutes.
99. The method according to clause 96, wherein the tissue growth medium comprises a bioabsorbable scaffold material.
100. The method according to clause 99, wherein the scaffold material comprises a skin substitute material.
101. The method according to clause 99, wherein the scaffold material comprises a collagen layer.
102. The method according to clause 96, wherein the tissue growth medium comprises a bone substitute material.
103. The method according to clause 102, wherein the bone substitute material comprises a bioabsorbable material.
104. The method according to clause 102, wherein the bone substitute material comprises at least one of calcium sulfate, calcium phosphate, a bioglass, or a ceramic.
105. A method for growing a tissue comprising the steps of:
   (a) applying a sub-atmospheric pressure to a tissue to be grown, wherein the applying step comprises the steps of:
      (i) placing a tissue growth medium in contact with a tissue to be grown;
      (ii) locating a cover over the tissue;
      (iii) sealing the periphery of the cover about the tissue; and
      (iv) operably connecting the cover with a vacuum system for producing the sub-atmospheric pressure; and
   (b) maintaining the sub-atmospheric pressure until the tissue has progressed toward a selected stage of growth within the tissue growth medium.
106. The method according to clause 105, wherein the maintaining of the sub-atmospheric pressure is conducted in alternating periods of application and non-application of the sub-atmospheric pressure.
107. The method according to clause 106, wherein each of the alternating periods is about 5 minutes.
108. The method according to clause 105, wherein the tissue growth medium comprises a bioabsorbable scaffold material.
109. The method according to clause 108, wherein the scaffold material comprises a skin substitute material.
110. The method according to clause 108, wherein the scaffold material comprises a collagen layer.
111. The method according to clause 105, wherein the tissue growth medium comprises a bone substitute material.
112. The method according to clause 111, wherein the bone substitute material comprises a bioabsorbable material.
113. The method according to clause 111, wherein the bone substitute material comprises at least one of calcium sulfate, calcium phosphate, a bioglass, or a ceramic.
114. A method for growing new bone tissue from existing bone tissue comprising the steps of:
   (a) placing a bone substitute material in contact with the existing bone tissue to provide a matrix in which to grow new bone tissue;
   (b) locating a cover over the existing bone tissue and the bone substitute material to form a sealed chamber at the bone tissue and the bone substitute material;
   (c) providing a subatmospheric pressure within the chamber; and
   (d) maintaining the sub-atmospheric pressure within the chamber until new bone tissue of a desired amount is grown within the bone substitute material.

## Claims

1. An apparatus for growing a tissue, comprising:
a. vacuum means for creating a sub-atmospheric pressure at a tissue to be grown;
b. sealing means operatively associated with the vacuum means for maintaining the sub-atmospheric pressure at the tissue by contacting the region surrounding the tissue; and
c. a tissue growth medium for positioning at the tissue within the sealing means and having an open-cell structure sufficiently porous to facilitate distribution of reduced pressure to the tissue to promote tissue growth within the porous tissue growth medium, the tissue growth medium comprising a bioabsorbable material including a naturally occurring fibrous material.

2. The apparatus of claim 1, wherein the tissue growth medium is perforated to enhance to enhance gas flow therethrough.

3. The apparatus of claim 1, wherein the tissue growth medium is not perforated.

4. The apparatus of any one of the preceding claims, wherein the tissue growth medium comprises collagen.

5. The apparatus of any one of the preceding claims, wherein the sealing means includes an adhesive-backed flexible polymer sheet for overlying the tissue growth medium to provide a seal over the tissue to be grown.

6. The apparatus of any one of the preceding claims, wherein the tissue growth medium comprises a fibrous-proteinaceous material.

7. The apparatus of any one of the preceding claims, wherein the tissue growth medium comprises a synthetic resorbable material.

8. The apparatus of any one of the preceding claims, wherein the tissue growth medium comprises a skin substitute material.

9. The apparatus of claim 8, wherein the skin-substitute material comprises a multilayer structure.

## Patentansprüche

1. Vorrichtung zum Züchten eines Gewebes, umfassend:
a. ein Vakuummittel zur Erzeugung eines subatmosphärischen Drucks an einem zu züchtendenden Gewebe;
b. ein Abdichtungsmittel, das operativ mit dem Vakuummittel verbunden ist, um den subatmosphärischen am Gewebe aufrechtzuerhalten, indem es mit dem das Gewebe umgebenden Bereich in Kontakt steht; und
c. ein Gewebewachstumsmedium zur Positionierung am Gewebe innerhalb des Abdichtungsmittels und mit einer offenzelligen Struktur, die ausreichend porös ist, um die Verteilung von reduziertem Druck am Gewebe zu erleichtern und so das Gewebewachstum innerhalb des porösen Gewebewachstumsmediums zu fördern, wobei das Gewebewachstumsmedium ein bioabsorbierbares Material umfasst, das ein natürlich vorkommendes faserförmiges Material umfasst.

2. Vorrichtung nach Anspruch 1, wobei das Gewebewachstumsmedium perforiert ist, um den Gasdurchfluss durch dieses zu fördern.

3. Vorrichtung nach Anspruch 1, wobei das Gewebewachstumsmedium nicht perforiert ist.

4. Vorrichtung nach einem der vorangegangenen Ansprüche, wobei das Gewebewachstumsmedium Collagen umfasst.

5. Vorrichtung nach einem der vorangegangenen Ansprüche, wobei das Abdichtungsmittel eine flexible Polymerfolie mit klebender Rückseite zum Darüberlegen über das Gewebewachstumsmedium umfasst, um eine Abdichtung über dem zu züchtenden Gewebe bereitzustellen.

6. Vorrichtung nach einem der vorangegangenen Ansprüche, wobei das Gewebewachstumsmedium ein faserförmiges, proteinartiges Material umfasst.

7. Vorrichtung nach einem der vorangegangenen Ansprüche, wobei das Gewebewachstumsmedium ein synthetisches, resorbierbares Material umfasst.

8. Vorrichtung nach einem der vorangegangenen Ansprüche, wobei das Gewebewachstumsmedium ein Hautersatzmaterial umfasst.

9. Vorrichtung nach Anspruch 8, wobei das Hautersatzmaterial eine mehrschichtige Struktur umfasst.

## Revendications

1. Appareil pour la croissance d'un tissu, comprenant :
a. un moyen permettant d'établir un vide pour créer une pression sous-atmosphérique à un tissu à cultiver ;
b. un moyen d'étanchéité fonctionnellement associé au moyen permettant d'établir un vide pour maintenir la pression sous-atmosphérique au tissu par une mise en contact avec la région entourant le tissu ; et
c. un support de croissance de tissu pour le positionnement au tissu dans le moyen d'étanchéité et ayant une structure à alvéoles ouvertes suffisamment poreuse pour faciliter la distribution de la pression réduite au tissu pour promouvoir la croissance du tissu dans le support de croissance de tissu poreux, le support de croissance de tissu comprenant un matériau bio-absorbable incluant un matériau fibreux se produisant naturellement.

2. Appareil selon la revendication 1, dans lequel le support de croissance de tissu est perforé pour encourager l'écoulement de gaz à travers celui-ci.

3. Appareil selon la revendication 1, dans lequel le support de croissance de tissu n'est pas perforé.

4. Appareil selon l'une quelconque des revendications précédentes, dans lequel le support de croissance de tissu comprend du collagène.

5. Appareil selon l'une quelconque des revendications précédentes, dans lequel le moyen d'étanchéité comprend une feuille de polymère flexible à endos adhésif destinée à recouvrir le support de croissance de tissu afin de réaliser un joint sur le tissu à cultiver.

6. Appareil selon l'une quelconque des revendications précédentes, dans lequel le milieu de croissance de tissu comprend un matériau fibreux- protéinique.

7. Appareil selon l'une quelconque des revendications précédentes, dans lequel le support de croissance de tissu comprend un matériau synthétique résorbable.

8. Appareil selon l'une quelconque des revendications précédentes, dans lequel le support de croissance de tissu comprend un matériau de substitution de la peau.

9. Appareil selon la revendication 8, dans lequel le matériau de substitution de la peau comprend une structure multicouche.
